# EUROPEAN PATENT APPLICATION

(11) **EP 3 992 300 A1**
(43) Date of publication of application: **04.05.2022**
(21) Application number: 20205016.7
(22) Date of filing: 30.10.2020
(51) Int. Cl.: C12Q 1/6806

(54) **SAMPLE PRE-TREATMENT FOR ISOTHERMAL AMPLIFICATION**

(71) Applicant: Nederlandse Organisatie voor toegepast- natuurwetenschappelijk Onderzoek TNO, 2595 DA 's-Gravenhage (NL)
(72) Inventor: KEIJSER, Bart Jan Frederik, 2595 DA TMs-Gravenhage (NL); OSSENDRIJVER, Michel, 2595 DA 's-Gravenhage (NL)
(74) Representative: V.O.

(57) **Abstract**

The invention is directed to a method for amplifying and/or detecting nucleic acid in a sample, comprising a specific pre-treatment step. In particular, the invention provides a pre-treatment for the LAMP method, which can further enhance the sensitivity of LAMP tests. The pre-treatment comprises a heat-treatment wherein the sample is heated in the presence of tris(2-carboxyethyl)phosphine (TCEP) and proteinase K.

## Description

### Field

The invention is directed to a method for amplifying and/or detecting nucleic acid in a sample, comprising a specific pre-treatment step. The invention is further directed to a kit for such methods.

### Introduction

Fast detection of infectious agents is important for monitoring, spreading, and identifying subjects infected with an infectious disease.

Currently, most virus detection tests are based on amplifying genetic material using the Polymerase Chain Reaction (PCR) method. These PCR tests are reliable, but also time-consuming and expensive for large-scale application. Furthermore, shortage of supplies of different components necessary to perform this test may limit seriously limit testing capacity. Therefore, there is a need for alternative amplification methods.

One of these alternative methods is the Loop-Mediated Isothermal Amplification (LAMP) method. This method also amplifies genetic material, but is based on a different principle of amplification, and requires different components than the PCR test. A significant advantage of LAMP over PCR is the increased speed with which the test can be conducted.

M. Lalli et al describe in the article *"*Rapid and extraction-free detection of SARS-CoV-2 from saliva with colorimetric LAMP" (https://doi.org/10.1101/2020.05.07.20093542) a LAMP-based assay for the qualitative detection of SARS-CoV-2 virus directly from saliva without an RNA extraction step. Several approaches were demonstrated to improve viral RNA detection in saliva samples. Lalli subjected the saliva sample to (1) a dilution step with water, or (2) a heat treatment step of 55°C for 15 minutes followed by 98°C for 5 minutes; or (3) the same heat treatment step in the presence of proteinase K. Lalli found that the heat treatment step improved improved SARS-CoV-2 particle detection in undiluted human saliva samples. Lalli further found that the presence of proteinase K in the heat treatment improved assay sensitivity compared to heat alone (see Lalli, page 6, 2nd paragraph). Lalli concludes that despite requiring brief inactivation at 98°C, proteinase K treatment to release viral RNA and inactivate ribonucleases and other inhibitors in saliva worked well to increase sensitivity in both RT-LAMP and qRT-PCR.

There is a need to provide sample pre-treatments for the LAMP method, which can further enhance the sensitivity of LAMP tests.

### Summary

In one aspect, the invention is directed to a pre-treatment of a sample comprising nucleic acid, wherein the sample is subjected to a heat-treatment in the presence of tris(2-carboxyethyl)phosphine (TCEP) and proteinase K.

In a further aspect, the invention is directed to a method for amplifying nucleic acid, comprising the steps of (i) providing a sample comprising nucleic acid; and (ii) the pre-treatment as described in the previous aspect, and further (iii) an amplification step, wherein the heat-treated sample is subjected to an amplification step, e.g. loop-mediated isothermal amplification.

In a further aspect, the invention is directed to a method for detecting the presence of a certain nucleic acid in a sample, which method comprises steps (i), (ii) and (iii) described above.

In a further aspect, the invention is directed to a kit suitable for conducting a method according to any one of the aspects above, wherein the kit comprises (i) a buffer comprising TCEP; and (ii) a proteinase K solution.

The inventors found that the pre-treatment according to the invention may enhance one or more of the following processes: (1) inactivation of enzymes present in the sample, such as RNase; and (2) removal of inhibitory factors (such as removal of proteins binding to enzymes in the diagnostic reaction); and (3) lysis of the shell enclosing the genetic material (such as the capsid of a viroid, a cell envelope, a cell membrane or a cell wall). Without wishing to be bound by any theory, it is expected that the pre-treatment enhances two or more or even all three of these processes. As a result, a lower amount of nucleic acid needs to be present in the sample in order to perform successful amplification. In particular, the pre-treatment of the invention will enhance the sensitivity of methods for detecting the presence of a certain nucleic acid, such as LAMP tests.

The combination of TCEP with Proteinase K surprisingly increases sensitivity of detecting nucleic acids compared to the use of TCEP or Proteinase K individually. Without being bound by any theory, the inventors believe that this is increase in sensitivity is due to a synergistic interaction between these two components in removing inhibitory components and/or inactivating RNase activity. Further, the effects of TCEP and/or Proteinase on lysis of the shell may play a role in the increase in sensitivity.

### Detailed description

The sample that is to be subjected to heat-treatment, amplification and detection is a sample comprising nucleic acids.

The sample may be an aqueous sample comprising the nucleic acids to be amplified. The sample may be taken from a mammal, e.g. from the human body. The sample may for example be a nasopharyngeal swab, an oral swab, a saliva sample, a blood sample, fecal sample, skin swab, biopsy, sputum sample, bronchial alveolar lavage (BAL) sample, vaginal swab or tissue sample. A preferred type of example is a nasopharyngeal swab sample. However, other samples are expected to work good as well, such as saliva samples or oral swabs. The nucleic acid will typically be present in the sample as part of a viroid particle or as part of a cell.

According to methods of the invention, a pre-treatment is conducted wherein the sample is subjected to a heat-treatment in the presence of tris(2-carboxyethyl)phosphine (TCEP) and proteinase K, and preferably also in the presence of ethylenediaminetetraacetic acid (EDTA). The heat-treatment according to the invention causes the nucleic acid to be released from the viroid particle or micro-organism in which it was enclosed. Furthermore, the heat-treatment may inactivate certain proteins. TCEP is capable of breaking disulphide bonds in proteins. Accordingly, TCEP may deactivate certain proteins in the sample, such as ribonuclease (RNase). By inactivating RNase, a smaller amount of RNA may be degraded between taking the sample and conducting amplification. Proteinase K is an enzyme which preferentially digests proteins after hydrophobic amino acids.

The pre-treatment may further comprise the step of preparing a mixture for the heat-treatment. Typically, this step comprises mixing the sample with TCEP and proteinase K. The resulting sample mixture is subjected to the heat-treatment. Before mixing, the sample may further be subjected to one or more other sample preparation steps, such as one or more of a dilution, filtration or purification step. However, such steps are not required for the invention to work, and are therefore preferably not performed.

The sample mixture that is to be subjected to the heat-treatment is preferably an aqueous mixture. The concentration TCEP in the sample mixture may be 0.1-100 mM, preferably 0.5-10 mM, more preferably 1-4 mM. The amount of proteinase K present in the sample mixture may be 0.005-1 wt.%, preferably 0.05-0.5 wt.%, more preferably 0.1-0.2 wt.% proteinase K. Furthermore, the sample mixture preferably comprises EDTA. The concentration EDTA in the sample mixture may be 0.05-50 mM, preferably 0.25-5 mM, more preferably 0.5-2 mM.

The weight ratio of proteinase K to TCEP in the sample mixture may lie between 1/2 and 8/1, preferably between 1/1 and 5/1. Good results have been obtained using a weight ratio between 2/1 and 4/1.

TCEP and EDTA can be added to the sample as part of a buffer. The concentration TCEP in the buffer may for example be in the range of 0.1 - 100 mM, preferably in the range of 1-10.

Preferably, the buffer further comprises ethylenediaminetetraacetic acid (EDTA). EDTA is preferably present in the buffer in a concentration of 0.05-100 mM, preferably 0.5-10 mM..

The heat-treatment is further conducted in the presence of proteinase K. The proteinase K may be added to the sample mixture as part of an aqueous solution Proteinase K may be present in the aqueous solution in an amount of 0,1 to 10 wt.%, for example 0,05 to 5,0 wt.%, based on the total weight of the buffer. Proteinase K is classified under Enzyme Commission (E.C.) number 3.4.21.64.

TCEP, Proteinase K, and EDTA are all soluble water. Accordingly, these compounds will be present during the heat-treatment in their dissolved form. TCEP may for example be provided in the sample mixture as a dissolved salt, for example as hydrochloride salt (TCEP-HCl). EDTA may be provided in the sample mixture as a dissolved salt, for example as disodium EDTA, calcium disodium EDTA, or tetrasodium EDTA.

The heat-treatment may comprise a first heating step, wherein the sample mixture is heated to a first temperature, and a second heating step, wherein the sample mixture is heated to a second temperature. In both heating steps, the sample mixture is heated in a buffer as described above. The buffer used in the first and second heating steps is preferably the same. Apart from the difference in temperature, the two sub-steps are preferably also conducted under similar (or even the same) reaction conditions.

The first heating step is preferably conducted at a temperature in the range of 40-75 °C, preferably 45-65 °C, more preferably 50-60 °C. The duration of the first heating step should not be too short. Preferably, the first heating step may be conducted for at least 3 minutes, preferably for 5-20 minutes, even more preferably for 7-15 minutes. Proteinase K is an enzyme which preferentially digests proteins at the C-terminus of hydrophobic amino acids. Elevated temperatures may provide proteinase K with improved access to these sites.

The second heating step may be conducted at a temperature in the range of 80-120 °C, preferably 90-105 °C, more preferably 95-100 °C. Such elevated temperatures will cause certain proteins in the sample to at least partially unfold, thereby making the proteins accessible to TCEP. Elevated temperatures of around 98 °C may be especially suitable for this purpose. In view of the stability of the buffer components, the duration of the second heating step should not be too long. Preferably, the second heating step is conducted for 10 minutes or less, more preferably for 30s to 8 minutes, even more preferably for 1-6 minutes.

The sequence in which the two heating steps are conducted is not particularly critical. The indication of a first and a second step are only used herein to distinguish between the two steps. Accordingly, either the first or the second heating step may be performed first in time. Both embodiments are within the scope of the invention. The heat-treatment may be conducted at a pH of 7 - 9, *e.g.* a pH of 7.5 - 8.5, such as a pH of 7.5 - 8.

The mixture obtained in the heat-treatment is referred to as the heat-treated sample.

The pre-treatment of the invention may further comprise the step of purifying the a heat-treated sample. Purification may make the heat-treated sample more suitable for amplification. For example, the heat-treated sample may be subjected to RNA enrichment. RNA enrichment may be conducted by contacting the heat-treated sample with magnetic beads that are capable of selectively binding RNA. In a subsequent step, RNA can be released from the magnetic beads, thereby obtaining an enriched heat-treated sample. In this way, essentially only the RNA from the original heat-treated sample will be present in the enriched heat-treated sample. RNA can be released from the magnetic beads by using an extraction buffer which releases RNA from the magnetic beads, followed by binding the magnetic beads to a magnet.

The pre-treatment according to the invention can be used as part of a method for amplifying nucleic acids. The pre-treatment provides a heat-treated sample that can be more effectively amplified, e.g. using the LAMP process.

The amplification method used is preferably loop-mediated isothermal amplification (LAMP). Unlike the aforementioned PCR method, LAMP does not require thermal cycles. LAMP is performed at a constant temperature. The skilled person will know which temperature to use based on the enzymes used in the LAMP process. In case of detecting SARS-CoV-2, LAMP is typically conducted in a temperature range of 60 - 65 °C.

The skilled person will know how to conduct an amplification method and which reaction conditions to use. For example, a suitable way of conducting the LAMP method is described in EP1020534 and EP1327679A1.

The polymerase used for amplification is not critical. The DNA polymerase may be a polymerase from Bacillus stearothermophilus or a homologue thereof. The skilled person will know what polymerases are suitable to conduct LAMP. A suitable DNA polymerase is for example described in WO2013/033528A1.

In a preferred embodiment, the amplification method is reverse transcription loop-mediated isothermal amplification (RT-LAMP). The RT-LAMP method is suitable for amplifying single-stranded nucleic acids. As such, it is very suitable for amplifying viral RNA.. RT-LAMP combines the LAMP DNA-detection with reverse transcription, making cDNA from single-stranded RNA or single-stranded DNA before amplification.

The pre-treatment and amplification method of the invention can be used in methods for detecting the presence of a certain nucleic acid in a sample. The inventors found that very high sensitivity can be achieved for detection of SARS-CoV-2 in an aqueous sample. The primers used for SARS-CoV-2 detection may be selected from 6-F3, 6-B3, 6-FIP, 6-BIP, 6-LOOPF and 6-LOOPB.

The amplification method of the invention will typically be conducted using an amplification reaction mixture, which mixture comprises the (optionally enriched) heat-treated sample, one or more primers, and a DNA polymerase. The primers used for amplification may be the five primers as defined in claim 1 of EP2031058B1. Specifically, these primers are a) a first primer that (i) provides at its 3'-end a starting point for complementary strand synthesis to a region that defines the 3' side of one of the strands that compose the target nucleotide sequence, and (ii) has on its 5'-side a nucleotide sequence that is complementary to an arbitrary region of a complementary strand synthesis reaction product that uses this primer as a starting point; b) a second primer that (i) anneals at its 3' end to the 3'-side region of a target nucleotide sequence on an elongation product of the first primer, and (ii) has on its 5'-side a nucleotide sequence that is complementary to an arbitrary region of a complementary strand synthesis reaction product of this primer, wherein the 3' end of the second primer provides a starting point for complementary strand synthesis; c) a first outer primer that provides a starting point for complementary strand synthesis to the 3'-side of a template of the first primer; d) a second outer primer that provides a starting point for complementary strand synthesis to the 3'side of a template of the second primer; and e) at least one of a first loop primer that provides a starting point for complementary strand synthesis using an elongation product of the first primer as a template, wherein the starting point of the first loop primer is different from the starting point of the first and second primers, and the starting point of the first loop primer is between a region corresponding to the first primer sequence in the elongation product of the first primer and the arbitrary region which anneals to the first primer, and a second loop primer that provides a starting point for complementary strand synthesis using an elongation product of the second primer as a template, wherein the starting point of the second loop primer is different from the starting point of the first and second primers, and the starting point of the second loop primer is between a region corresponding to the second primer sequence in the elongation product of the second primer and the arbitrary region which anneals to the second primer. In this case, the reaction mixture may further comprise a DNA polymerase that catalyzes complementary strand synthesis with accompanying strand displacement; and a substrate for complementary strand synthesis.

Detection of the nucleic acid may for example be fluorescence based. In this embodiment, a fluorescent nucleic acid stain may be added to the amplification reaction mixture. As the amount of nucleic acid increases in the reaction mixture, the fluorescent signal will become stronger.

Amplification and detection may be conducted simultaneously. Alternatively, detection may be conducted after amplification. Suitable detection methods are known to the skilled person. In case of fluorescence based detection, detection may be conducted by measuring fluorescence (e.g. using a fluorometer).

The nucleic acid present in the sample may be RNA or DNA. The nucleic acid can be single-stranded or double-stranded. In case the nucleic acid is single-stranded, reverse transcription loop-mediated isothermal amplification (RT-LAMP) can be suitably used as the amplification method. As shown in the experimental example further below, the effect on removing inhibitory components and/or inactivating RNase activity was observed to be strong in experiments using RNA as the nucleic acid.

The nucleic acid may be viral RNA or viral DNA. In case the nucleic acid is single-stranded viral RNA, the RNA may be from a virus that belongs to Group IV or V of the Baltimore Virus Classification system. Examples of viruses with single-stranded RNA are coronaviruses, such as SARS coronavirus and MERS coronavirus. In case the genetic material is single-stranded viral DNA, the DNA may be from a virus that belongs to group II in the Baltimore Virus Classification system.

The nucleic acid may be from a virus selected from SARS-CoV-2, SARS-CoV-1, MERS-CoV, influenza virus and Ebola virus. The nucleic acid to be amplified may be bacterial nucleic acid. For example the nucleic acid may be from a bacteria selected from the group consisting of *Mycobacterium tuberculosis, Corynebacterium diphtheriae,* and *Bordetella pertussis.*

The nucleic acid to be amplified may be from a micro-organism, in particular from a single-celled micro-organisms. For example, the nucleic acid may be from a micro-organism that causes malaria, such as micro-organisms of the Plasmodium group, *e.g.* one selected from *Plasmodium falciparum, Plasmodium vivax, Plasmodium ovale,* and *Plasmodium malariae.* The nucleic acid may also be from a micro-organism that causes sleeping sickness, such as micro-organisms of *Trypanosoma brucei,* such as *Trypanosoma brucei gambiense* (TbG) and *Trypanosoma brucei rhodesiense* (TbR)

The infectious disease may be selected from SARS-CoV-2, SARS-CoV-1, tuberculosis, malaria, sleeping sickness and influenza.

In a further aspect, the invention is directed to a kit suitable for conducting a method according to one or more of the aspects above, wherein the kit comprises the buffer described above, proteinase K, and optionally a DNA polymerase and one or more LAMP primers for nucleic acid detection.

In a preferred embodiment, the method of the invention is used to amplify and/or detect SARS-CoV-2. In this embodiment, the sample is preferably a nasopharyngeal swab. The buffer comprises TCEP, Proteinase K and preferably also EDTA. The heat-treatment comprises a first heating step at 50-60 °C and a second heating step at 90-100 °C. The primer set used for amplification preferably comprises at least 4, more preferably at least 5 of the group of primers consisting of 6-F3, 6-B3, 6-FIP, 6-BIP, 6-LOOPF and 6-LOOPB.

The invention will now be further illustrated by the following non-limiting example.

### Example:

### Preparation of the Buffer:

First, a 0.5 M TCEP-HCl solution was prepared by dissolving 358 mg (1.25 mmol) tris(2-carboxyethyl)phosphine hydrochloride (TCEP-HCl) in 2.5 ml water. Further, a 0.5 M EDTA solution was prepared by first dissolving 1.461 of a ethylenediaminetetraacetic acid disodium salt dihydrate in 2 ml water, and supplementing the solution thus obtained with 4M NaOH and water to a pH of 8.0 and a total volume of 10 ml. An amount of 1 ml of the 0.5 M EDTA solution was then added to the 2.5 ml TCEP-HCl solution. To the TCEP/EDTA solution thus obtained, an amount of 575 µl 10M NaOH and 925 µl water was added to create a 5 ml buffer with 0.25 M TCEP, 0.1 M EDTA, and pH 9.

### Proteinase K solution:

A solution of proteinase K in water was obtained from Sigma Aldrich which had a concentration of 1.8 wt.% proteinase K (18 mg proteinase K per 1 ml).

### Preparation of virus particle solution:

A stock solution with a known amount of SARS-CoV-2 virus particles (2x10⁹ copies/ml) was used to prepare the sample. An amount of 5 µl stock solution was first diluted with phosphate-buffered saline (1000-fold) to create a solution of 10⁴ virus particles, and subsequently further diluted with universal transport medium (10-fold) to create a 100 µl sample containing 1000 virus particles.

### Heat-Treatment:

Four different sample mixtures were prepared by mixing different amounts of buffer, Proteinase K solution and sample. Sample mixture 1 contained both buffer and proteinase K solution, while sample mixtures 2 and 3 only contained only one of these two components. Sample mixture 4 did neither contain buffer nor proteinase K solution. The specific amounts of components used are shown in Table 1 below.

**Table 1: Sample Mixture Composition**

| | Virus Sample (µl) | TCEP/EDTA Buffer (µl) | Proteinase K solution (µl) |
|---|---|---|---|
| Sample Mixture 1 | 100 | 1 | 10 |
| Sample Mixture 2 | 100 | 1 | 0 |
| Sample Mixture 3 | 100 | 0 | 10 |
| Sample Mixture 4 | 100 | 0 | 0 |

Each mixture was prepared six times. Each mixture was subjected to one of three heat-treatments:
- Heat-Treatment A: heating the mixture for 5 minutes at 95 °C
- Heat-Treatment B: heating the mixture for 10 minutes at 55 °C
- Heat-Treatment C: heating the mixture for 10 minutes at 55 °C followed by heating the mixture for 5 minutes at 95 °C.
After the heat-treatment, the mixtures were cooled on ice for 5 minutes. The mixtures obtained in the step are called the heat-treated samples.

The different heat-treated samples thus obtained are shown in Table 2.

Each sample mentioned in the table was prepared twice (for a total of 24 heat-treated samples).

**Table 2: Heat-Treated Samples**

| | Heat-Treatment A (5 min at 95 °C) | Heat-Treatment B (10 min at 55 °C) | Heat-Treatment C (10 min at 55°C; 5 min at 95 °C) |
|---|---|---|---|
| Sample Mixture 1 (TCEP+Proteinase K) | Sample 1A | Sample 1B | Sample 1C |
| Sample Mixture 2 (TCEP) | Sample 2A | Sample 2B | Sample 2C |
| Sample Mixture 3 (Proteinase K) | Sample 3A | Sample 3A | Sample 3C |
| Sample Mixture 4 (none) | Sample 4A | Sample 4B | Sample 4C |

### RNA Purification:

The heat-treated sample was subjected to a purification step, which makes use of magnetic beads that are capable of selectively binding RNA. A high-salt buffer with pH 8 was prepared, which buffer comprised 2.5M sodium chloride (NaCl); 10 mM 2-amino-2-(hydroxymethyl)-1,3-propaandiol, hydrochloride (Tris-HCl); 20% (w/v) polyethylene glycol (PEG8000); 0.05% nonionic detergent (Tween 20); and 5mM NaN₃. An amount of 200 µl magnetic beads capable of selectively binding RNA (RNAClean XP; Beckman) was diluted with 1 ml of the high-salt buffer (1:5 dilution). An amount of 200 µl magnetic beads were diluted with 1ml high-salt buffer (1:5 dilution). Subsequently, 60 µl beads were mixed with 100 µl of the heat-treated sample and then incubated for 10 minutes at 20 °C. The solution thus obtained was incubated for 5 minutes on the magnet upon which the beads formed a pellet. Subsequently, the supernatant was removed and the pellet was washed twice with 85% ethanol and then dried at 50 °C to remove the ethanol.

### Amplification and Detection:

The heat-treated samples were subjected to reverse transcription loop-mediated isothermal amplification (RT-LAMP).

The following primers were used (with the relevant DNA sequence added in brackets):
- 6-F3 (CGGTGGACAAATTGTCAC)
- 6-B3 (CTTCTCTGGATTTAACACACTT)
- 
6-FIP (TCAGCACACAAAGCCAAAAATTATTTTCTGTGCAAAG
GAAATTAAGGAG)
- 
6-BIP (TATTGGTGGAGCTAAACTTAAAGCCTTTTCTGTACAATC
CCTTTGAGTG)
- 6-LOOPF (TTACAAGCTTAAAGAATGTCTGAACACT)
- 6-LOOPB (TTGAATTTAGGTGAAACATTTGTCACG)

The DNA polymerase used was a homologue of the Bacillus stearothermophilus DNA Polymerase (Bst 2.0 WarmStart^{®} DNA Polymerase, obtained from New England Biolabs).

RNA Detection was conducted simultaneously with amplification. For this purpose, a fluorescent nucleic acid stain (SYTO 9) was used.

The LAMP reaction mixture was prepared by mixing the primer solutions, RNase solution, purified heat-treated samples, and fluorescent nucleic acid stain together. The reaction mixture before the LAMP reaction showed a pink/purple color.

The LAMP reaction was conducted for 10 minutes at a temperature of 64-65 °C. One cycle was defined as 15 seconds. Accordingly, the LAMP reaction was conducted for 40 cycles. The LAMP reaction was stopped by heating the reaction mixture at 85°C for 2 minutes to deactivate the DNA polymerase.

During the LAMP reaction, the reaction mixtures showed a color change from pink/purple to yellow after a certain amount of time. This change in color is caused by the fluorescent nucleic acid stain, which is built in the amplified RNA. The change in color provides a visual indication that the amount of SARS-CoV-2 exceeds a certain threshold concentration.

The presence of a detectable amount of SARS-CoV-2 RNA in the LAMP reaction was measured by an apparatus for measuring fluorescence (*e.g.* a fluorometer, in this case an Applied Biosystems 7500 qPCR machine was used). The apparatus will detect a fluorescent signal if a sufficient amount of fluorescent RNA is present in the LAMP reaction mixture. Since each heat-treated sample contained the same amount of virus particles, the quality of the heat-treatment of the sample will determine how quickly a fluorescent signal can be measured (as well as how quickly the reaction mixture will change color).

The amount of time it took for each reaction mixture to change color was expressed in the number of cycles (1 cycle = 15 seconds) and is shown in Table 3. Each combination of sample and heat-treatment was conducted twice, but the result for the same combination was each time the same.

The heat-treatment according to the invention (sample mixture 1 + heat-treatment C) only required 17 cycles before the color change occurred.

**Table 3: Number of cycles needed for the color change to occur in the LAMP reaction for the different sample / heat-treatment combinations:**

| | Heat-Treatment A (5 min at 95 °C) | Heat-Treatment B (10 min at 55 °C) | Heat-Treatment C (10 min at 55°C; 5 min at 95 °C) |
|---|---|---|---|
| Sample Mixture 1 (TCEP+Proteinase K) | 37 cycles | no color change within 40 cycles | 17 cycles |
| Sample Mixture 2 (TCEP) | 39 cycles | 29 cycles | no color change within 40 cycles |
| Sample Mixture 3 (Proteinase K) | no color change within 40 cycles | no color change within 40 cycles | no color change within 40 cycles |
| Sample Mixture 4 (none) | no color change within 40 cycles | 20 cycles | no color change within 40 cycles |

### Conclusion:

By using the heat-treatment according to the invention, it is possible to inactivate the sample to such an extent, that the LAMP test will already show a detectable amount of RNA in 17 cycles. This is a surprisingly low amount of cycles, especially considering that a heat-treatment with only TCEP (sample mixture 2) or with only Proteinase K (sample mixture 3) always required 29 cycles or more.

A LAMP test will be considered sufficiently sensitive to detect SARS-CoV-2 if it can detect the presence of the viral RNA within 30 cycles of 15 seconds. The heat-treatment according to the invention thus makes it possible that LAMP can be used with sufficient accuracy to determine a positive SARS-CoV-2 test.

Another surprising result was the 20 cycles it took for the sample without buffer or proteinase K (sample mixture 4) after heat-treatment B to be detected. However, it is not desirable to conduct LAMP on a sample without inactivation buffer, as it would be considered unsafe for practical application since it does not disrupt virus particles.

## Claims

1. A method for amplifying nucleic acid, comprising the steps of
(i) providing a sample comprising nucleic acid; and
(ii) subjecting the sample to a heat-treatment in the presence of tris(2-carboxyethyl)phosphine (TCEP) and proteinase K; and
(iii) an amplification step, wherein the heat-treated sample is subjected to loop-mediated isothermal amplification.

2. The method according to claim 1, wherein the sample is subjected to a heat-treatment in the presence of tris(2-carboxyethyl)phosphine (TCEP), proteinase K, and ethylenediaminetetraacetic acid (EDTA).

3. The method according to claim 1, wherein the nucleic acid is viral RNA.

4. The method according to claim 3, wherein the nucleic acid is SARS-CoV-2.

5. The method according to any of the previous claims, wherein the sample is a saliva sample or a nasopharyngeal swab.

6. The method according to any of the previous claims, further comprising the step of preparing a sample mixture by mixing the sample with TCEP and proteinase K; and subjecting the sample mixture to said heat-treatment.

7. The method according to claim 6, wherein the concentration TCEP in the sample mixture is 0.5-10 mM, preferably 1-4 mM and the amount of proteinase K in the sample mixture is 0.05-0.5 wt.%, preferably 0.1-0.2 wt.%.

8. The method according to any of the previous claims, the weight ratio of proteinase K to TCEP in lies between 1/2 and 8/1, preferably between 1/1 and 5/1, more preferably between 2/1 and 4/1.

9. The method according to any of the previous claims, wherein the heat-treatment comprises
- a first heating step, wherein the sample or sample mixture is heated to a first temperature of 45-65 °C for 5-20 minutes; and
- a second heating step, wherein the sample or sample mixture is heated to a second temperature of 90-100 °C for 1-8 minutes,

10. The method according to any of the previous claims, wherein the heat-treated sample is subjected to reverse transcription loop-mediated isothermal amplification (RT-LAMP)

11. A method for detecting viral RNA from a sample, comprising the steps of
(i) providing a sample comprising nucleic acid; and
(ii) subjecting the sample to a heat-treatment in the presence of tris(2-carboxyethyl)phosphine (TCEP) and proteinase K; and
(iii) an amplification step, wherein the heat-treated sample is subjected to loop-mediated isothermal amplification; and
(iv) detecting the amplified viral RNA.

12. A pre-treatment for inactivating a sample comprising nucleic acid, comprising subjecting the sample to a heat-treatment in the presence of tris(2-carboxyethyl)phosphine (TCEP) and proteinase K.

13. A kit for for treating a sample comprising nucleic acid to be amplified, comprising
- a buffer comprising TCEP and optionally EDTA; and
- a proteinase K solution; and
- optionally a DNA polymerase and/or one or more LAMP primers.
